# EUROPEAN PATENT APPLICATION

(11) **EP 4 495 261 A1**
(43) Date of publication of application: **22.01.2025**
(21) Application number: 23306255.3
(22) Date of filing: 20.07.2023
(51) Int. Cl.: C12Q 1/6883

(54) **DETECTION OF CORTICOSTEROID EFFICIENCY BY ANALYSING THE RS37973 AND RS28362491 POLYMORPHISMS**

(71) Applicant: Université de Versailles Saint-Quentin-en-Yvelines, 78035 Versailles Cédex (FR); Assistance Publique - Hôpitaux de Paris, 75012 Paris (FR); Institut National de la Santé et de la Recherche Médicale, 75013 Paris (FR)
(72) Inventor: FLEURIET, Jérôme, 92420 VAUCRESSON (FR); GARCHON, Henri-Jean, 92140 CLAMART (FR); MAMBU MAMBUENI, Hendrick, 78390 BOIS-D'ARCY (FR); MSLMANE, Alaa, 78180 MONTIGNY LE BRETONNEUX (FR); ANNANE, Djillali, 94700 MAISON ALFORT (FR)
(74) Representative: Regimbeau

(57) **Abstract**

Corticosteroids are currently the only available treatment that provides some benefits to selected patients with septic shock. However, severe side-effects on mortality of hydrocortisone therapy have been reported and there is still an uncertainty of the therapeutic relevancy of hydrocortisone therapy in a number of patients. In this context, the present inventors identified two polymorphic biomarkers that are associated with corticosteroid efficiency independently or in combination. These two polymorphisms, rs2836249 and rs37973, can be detected in patients' blood rapidly and easily, to determine if a corticosteroid treatment would be valuable or inactive in these patients. The present invention therefore provides personalized treatment and diagnostic companion to help establishing the best treatment options for patients suffering from sepsis and potentially from other conditions such as severe asthma, that are treatable with corticosteroids.

## Description

### Summary of the invention

Corticosteroids are currently the only available treatment that provides some benefits to selected patients with septic shock. However, severe side-effects on mortality of hydrocortisone therapy have been reported and there is still an uncertainty of the therapeutic relevancy of hydrocortisone therapy in a number of patients. In this context, the present inventors identified two polymorphic biomarkers that are associated with corticosteroid efficiency independently or in combination. These two polymorphisms, rs2836249 and rs37973, can be detected in patients' blood rapidly and easily, to determine if a corticosteroid treatment would be valuable or inactive in these patients. The present invention therefore provides personalized treatment and diagnostic companion to help establishing the best treatment options for patients suffering from sepsis and potentially from other conditions such as severe asthma, that are treatable with corticosteroids.

### Description of the prior art

Sepsis, an abnormal host response to an infection [1,2], is a public health priority owing to its annual incidence of over 49 million patients worldwide, a crude annual mortality of about 11 million deaths, and long-term sequels in about half of survivors [3,4]. The average cost of caring sepsis patient has been estimated to 16,000 $ in Western countries [5]. Corticosteroids help to control the dysregulated immune response causing sepsis and to increase blood pressure if it is low. Some clinicians have found this biological rationale, and results of early studies, compelling. Yet, others disagree and do not use corticosteroids, as the treatment may have no effect or even induce negative effects on some patients. In view of these contradictory studies, most professional organizations recommend against corticosteroid use in the absence of refractory shock. However, corticosteroids are currently the only available treatment that provides some benefits to patients suffering septic shock [6-9] and it would be prejudicial to exclude this treatment in critical cases.

Asthma is a prevalent chronic disease of the respiratory system and acute asthma exacerbations are among the most common causes of presentation to the emergency department (ED) and admission to hospital particularly in children. Inhaled corticosteroids (ICS), through their anti-inflammatory effects have been the mainstay of treatment of asthma for many years. Systemic and ICS are also used in the treatment of acute asthma exacerbations. Several international asthma management guidelines recommend the use of systemic corticosteroids in the management of moderate to severe acute asthma early upon presentation to the ED. On the other hand, ICS use in the management of acute asthma has been studied in different contexts with encouraging results in some and negative in others.

In this context, it is important to understand what causes the heterogeneity in patients' responses to corticosteroids. Given the heritability within the therapeutic class of glucocorticosteroids, as well as the high degrees of between-patient variability and within-patient repeatability in the response to glucocorticoids in some diseases, it has been proposed that this response should have a genetic basis. Accordingly, a number of pharmacogenetic investigations have been performed, focusing on several candidate biomarker genes. However, none of them is strong enough so far to reproducibly and efficiently predict if a corticosteroid treatment would fail or succeed in a patient in need thereof.

The present invention solves this problem.

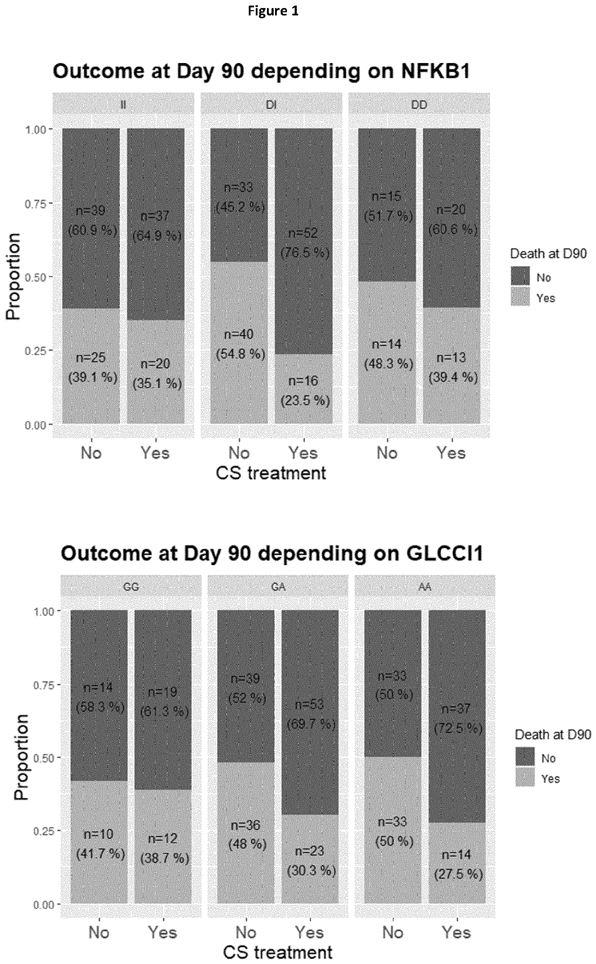

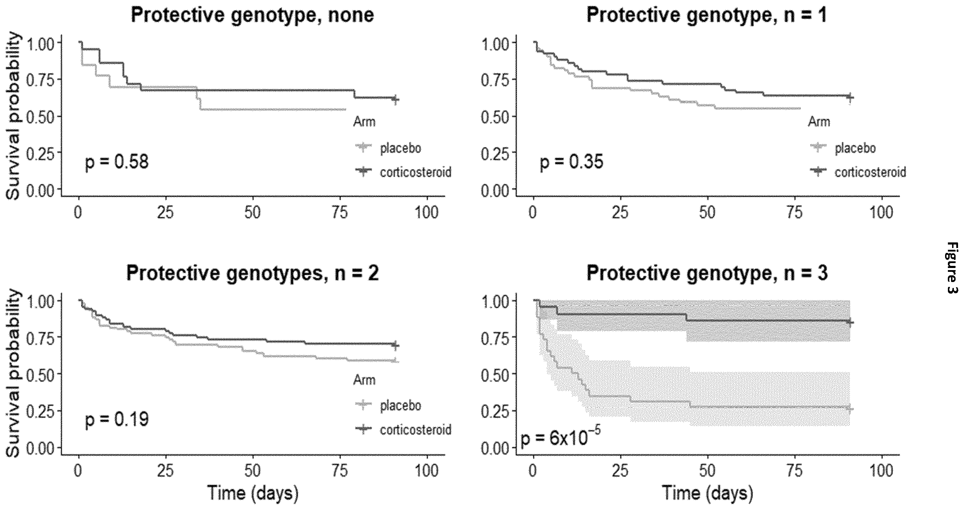

As a matter of fact, the present inventors discovered that the polymorphism rs28362491 located in the upstream region of the Nuclear Factor Kappa B subunit 1 (NFKB1) gene and the polymorphism rs37973 located in the promoter of the Glucocorticoid Induced 1 (GLCCI1) gene are independently good predictive factors of corticosteroid efficiency (figure 1). Moreover, they showed that the two polymorphisms could be advantageously associated to predict more efficiently the responsiveness to corticosteroids. As a matter of fact, in the present study, the inventors show that the presence of the two variants interacts with the effects of hydrocortisone plus fludrocortisone on mortality in the Activated Protein C and Corticosteroids for Human Septic Shock (APROCCHSS) cohort (figure 3) [8].

This is the first time that the heterozygous genotype DI for rs28362491 is proposed as a good prognosis marker of corticosteroid's response. Also, it is the first time that the heterozygous genotype GA for rs37973 is proposed as a good prognosis marker of corticosteroid's response. The prognostic use of these markers, separately or in combination, is therefore novel and inventive.

### Detailed description of the invention

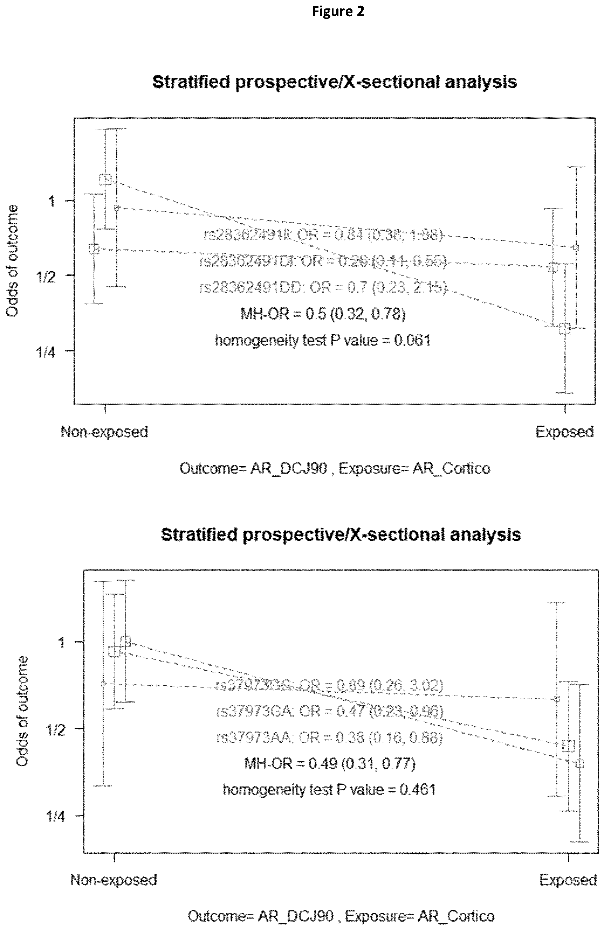

The rs28362491 variant is a known polymorphism located in the upstream region of the *Nuclear Factor Kappa B subunit 1 (NFKB1)* gene on chromosome 4q24 at position 102500898 of the GRCh38 build of the reference sequence of the human genome. It is also known in the early literature as "94ins/del ATTG", because it consists in the deletion of ATTG tetranucleotide sequence that was believed to locate at 94 nucleotides after the start of the transcription. If the ATTG is inserted, the allele is "I". If the ATTG is deleted, the allele is "D". It has been previously shown that D allele carriers undergoing hydrocortisone therapy had a more than 3-fold increased risk for death compared to allele carriers without corticosteroids therapy. Thus, the "D allele" was previously shown to be prejudicial to corticosteroid therapy (Schäfer et al, 2014 = [10]). Contrarily to this observation, the present results show that corticosteroid therapy is in fact beneficial in patients carrying the genotype DI in this rs28362491 variant (figures 1 and 2).

The rs37973 variant is also known in the art. It is located in the chromosome 7, in the promoter of *the Glucocorticoid Induced 1 (GLCCI1)* gene, e.g., in position 7968145 of the GRCh38 build of the reference sequence of the human genome. At this particular position, two alternative nucleotides have been detected : the wild-type A or the mutated G. It has been previously described that the presence of two mutated alleles GG reduces the efficiency of glucocorticoids and that the presence of two wild-type alleles A is associated with corticosteroid efficiency in patients with asthma (Tantisira et al. 2011 = [11]). The present results show that corticosteroid therapy is beneficial in patients carrying the heterozygous genotype GA in the rs37973 variant (figures 1 and 2).

### Treatment methods

The results of the inventors show that corticosteroids will only have a significant positive effect on the patient's survival if said patient carries the genotype DI of the rs28362491 variant or if said patient carries the genotype GA or AA in the rs37973 variant (figure 1). Moreover, they show that the effect of corticosteroids on patient's survival is surprisingly enhanced when the two variants are taken into account (figure 3). The present inventors therefore propose to combine the two prognosis markers rs28362491 and rs37973 to select patients that will mostly and more durably benefit from the corticosteroid treatment.

More precisely, the figures 1 and 3 disclose that, contrarily to what could have been predicted from the teaching of Schäfer et al, 2014 = [10], the presence of the allele DI for rs28362491 is a good prognosis factor and will ensure that the corticosteroid treatment is efficient. Figure 3 shows that this good prognosis factor is advantageously combined with rs37973, especially when the patient carries the AA allele at rs37973 (when n=3). And figure 5 (lower panel) shows that this good prognosis factor is also advantageously combined with the genotype GA of rs37973. In contrast, there was no corticosteroid effect in the patients lacking the rs28362491^{∗}DI genotype or carrying the homozygous rs37973^{∗}GG genotype (figure 6). Altogether, this means that it is most beneficial to administer a corticosteroid treatment to patients carrying the genotype DI of the rs28362491 variant and the genotype GA or AA in the rs37973 variant.

In a first aspect, the present invention thus relates to corticosteroids for use for treating a patient suffering from a disease treatable by corticosteroids, wherein said patient carries the genotype DI of the rs28362491 variant located in the *NFKB1* gene and the genotype GA or AA in the rs37973 variant located in the promoter of the *GLCCI1* gene.

[forme Suisse] The invention also encompasses the use of corticosteroids for the manufacture of a medicament intended for treating a patient suffering from a disease treatable by corticosteroids, wherein said patient carries the genotype DI of the rs28362491 variant located in the *NFKB1* gene and the genotype GA or AA in the rs37973 variant located in the promoter of the *GLCCI1* gene.

[forme US] Moreover, the invention encompasses a method for treating a patient suffering from a disease treatable by corticosteroids, said method comprising the step of administering corticosteroids to said patient only if he / she carries the genotype DI of the rs28362491 variant located in the *NFKB1* gene and the genotype GA or AA in the rs37973 variant located in the promoter of the *GLCCI1* gene.

In a preferred embodiment, said patient carries the genotype DI in rs28362491 and the genotype AA in rs37973. As a matter of fact, the results below show that corticosteroid therapy is most beneficial when it is administered in patients carrying the genotype DI in rs28362491 and the genotype AA in rs37973 (figure 3, when n=3).

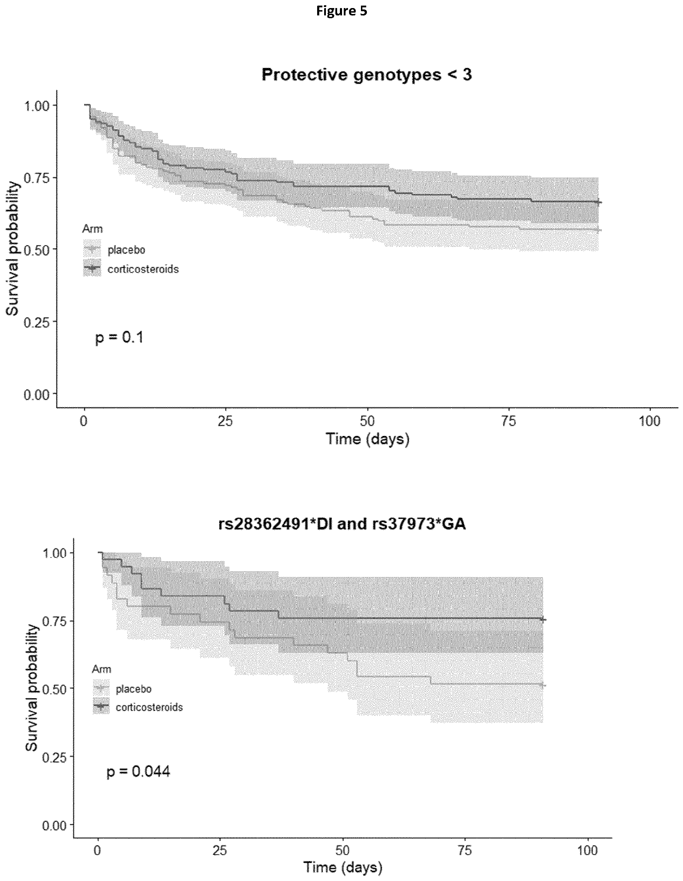

In another preferred embodiment, said patient carries the genotype DI in rs28362491 and the genotype GA in rs37973. As a matter of fact, the results below show that corticosteroid therapy is also beneficial when it is administered in patients carrying the genotype DI in rs28362491 and the genotype GA in rs37973 (figure 5, lower panel).

As used herein, a "disease treatable by corticosteroids" is any disease whose symptoms or etiology are known to be improved by a corticosteroid treatment. For example, said disease can be an allergy, an atopy, an inflammatory condition or a cancer.
- Allergies can be chosen among: Asthma (severe exacerbations), Allergic rhinitis, Atopic dermatitis, Urticaria, Angioedema, Anaphylaxis, Food allergies, Drug allergies, Nasal polyps, Hypersensitivity pneumonitis, Eosinophilic pneumonia, Contact dermatitis;
- Inflammatory conditions can be chosen among: Chronic obstructive pulmonary disease, Pemphigus vulgaris, Sarcoidosis, Behcet's disease, Interstitial lung disease, Ulcerative colitis, Crohn's disease, Autoimmune hepatitis, Autoimmune hemolytic anemia, Idiopathic thrombocytopenic purpura, Systemic lupus erythematosus, Polymyalgia rheumatica, Spondylarthropathies, Acquired myasthenia gravis, Polymyositis, Dermatomyositis, Polyarteritis, Vasculitis, systemic sclerosis, Sjögren's syndrome, Uveitis, Optic neuritis, Keratoconjunctivitis, Multiple sclerosis relapses, Organ transplantation, Nephrotic syndrome, Chronic hepatitis (flare ups), Cerebral edema, IgG4-related disease, Tendinosis, Lichen planus, Duchenne muscular dystrophy, acute respiratory distress syndrome (ARDS); and
- Cancer can be chosen among: Lymphomas, Leukemias, Multiple Myeloma, Prostate cancer. In a particular embodiment, the disease is preferably severe asthma or sepsis.

The present invention can involve the treatment of the patient with any corticosteroid or derivative thereof that has already been approved or that will be approved later. For example, the corticosteroid can be chosen in the group consisting of : hydrocortisone, hydrocortisone acetate and other esters, cortisone acetate, tixocortol pivalate, prednisolone, methylprednisolone, prednicarbate, prednisone, amcinonide, budesonide, desonide, fluocinolone acetonide, fluocinonide, flunisolide, fludroxycortide, halcinonide, deflazacort, ulobetasol, ciclesonide, triamcinolone acetonide, beclomethasone, betamethasone, dexamethasone, fluocortolone, halometasone, paramethasone, mometasone, alclometasone dipropionate, betamethasone dipropionate, betamethasone valerate, clobetasol propionate, clobetasone butyrate, fluprednidene acetate, and mometasone furoate, or any other hydrocortisone, acetonide, or betamethasone derivatives having the same biological function. In these therapeutic uses or methods, the detection of the rs variants can be performed by any conventional means, such as those described below.

### Prognosis methods

In another aspect, the present invention relates to an *in vitro* method for predicting the efficiency of a corticosteroid treatment, said method comprising the step of detecting the genotype of the rs28362491 variant and/or of the rs37973 variant in a biological sample of a patient in need thereof.

Given the ease of detecting specific nucleic acid sequences, this method may be performed at a very low cost. This method thus provides rapid and simple means of determining if a corticosteroid treatment will be efficient when administered to a patient in need thereof.

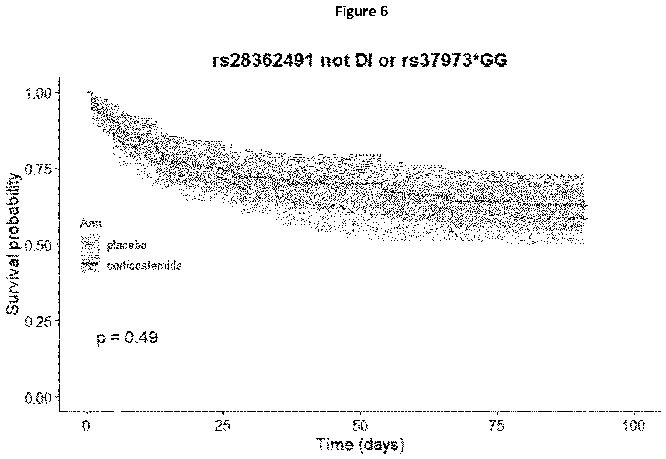

As a matter of fact, the inventors herein show that, if corticosteroids are administered to a patient that does not carry the genotype DI for rs28362491 or to a patient that carries the genotype GG for rs37973, then the corticosteroid treatment has no effect on the patient's outcome (figure 6). Conversely, when corticosteroids are administered to a patient carrying the genotype DI for rs28362491 (figure 1, upper panel) or GA for rs37973 (figure 1, lower panel) or to a patient that carries both genotypes (figure 5), then the corticosteroid treatment has a significant effect on the survival of the patient.

Also, it is shown in the enclosed data that the corticosteroid treatment will not be efficient if it is administered to a patient carrying the genotype DD or II in the rs28362491 variant (figure 1 upper panel) or the genotype GG in the rs37973 variant (figure 1, lower panel), or to those that do not carry DI nor AA (figure 5 upper panel).

In a preferred embodiment, it is concluded that a corticosteroid treatment will be efficient if the patient carries i) DI genotype of rs28362491 and/or ii) the GA genotype of rs37973.

In a more preferred embodiment, it is concluded that the corticosteroid treatment will be efficient if the patient carries i) the DI genotype of rs28362491 and ii) the GA genotype of rs37973 (figure 5 lower panel).

In another preferred embodiment, it is concluded that a corticosteroid treatment will be efficient, if the patient carries i) the DI genotype of rs28362491 and ii) the AA genotype of rs37973.

Thus, the present invention also concerns a method comprising the step of detecting the genotype of both the rs28362491 and the rs37973 variants in a biological sample of a patient in need thereof, wherein it is concluded that the corticosteroid treatment will be efficient in said patient if the rs28362491 variant is of the DI genotype and if the rs37973 variant is of the AA or GA genotype.

As a matter of fact, the inventors herein show that, if corticosteroids are administered to a patient that does not carry the genotype DI for rs28362491 or that carries the genotype GG for rs37973, then the corticosteroid treatment will have no effect on the patient (figure 6).

In a preferred embodiment, the "patient in need thereof" suffers from a "disease treatable by corticosteroids" as defined above, that is, any disease whose symptoms or etiology are known to be improved by a corticosteroid treatment. Typically, said disease can be an allergy, an atopy, an inflammatory condition or a cancer, or any particular conditions requiring a corticosteroid treatment, as disclosed above.

For the purposes of the present invention, the term "patient" herein means a mammal, preferably a human, irrespective of age. Thus, the patient may be for example an adult or a child. "Adult" means an individual who is at least 16 years of age. "Child" refers to an individual whose age is less than 16 years of age, particularly infants from birth to 1 year of age, and children from 1 to 15 years of age.

The expression "biological sample" as used herein refers to any sample comprising nucleic acids, obtained from a patient. A sample may comprise tissues and/or biological fluids. Such samples can be obtained *in vitro, ex vivo* or *in vivo.* As a non-limiting example, the biological sample may be selected from tissues, organs, cells, or any isolated fraction of a patient. The biological sample may also be selected from biological fluids including but not limited to blood, plasma, lymph, saliva, urine, stool, tears, sweat, sperm, or cerebrospinal, synovial, pleural, peritoneal, or pericardial fluid, as well as any fraction thereof.

In a preferred embodiment, said biological sample is whole blood or plasma or serum or saliva or hair or urine sample of said patient. In a most preferred embodiment, it is whole blood. The sample may also be pre-processed to preserve the integrity of the nucleic acids and/or to make them more accessible for further analysis. For example, the sample may be treated with anti-nucleases. The sample may also undergo lysis steps (e.g. chemical, mechanical, or enzymatic lysis), centrifugation, purification, etc. to facilitate access to nucleic acids and/or to concentrate them.

Said sample can be obtained by any technique known in the prior art. Said blood or plasma sample may be obtained by a completely harmless blood collection from the subject and thus advantageously allows for a non-invasive detection. The blood sample used in the method of the invention is preferably depleted of most, if not all, erythrocytes, using common red blood cell lysis procedures. Preferably, peripheral blood mononuclear cells are prepared from the blood sample. Said saliva sample may be obtained with a simple mouth swab or using the passive drool technique.

The term "detection" as used herein refers to any means allowing for the identification of the variants / polymorphisms of the invention (rs28362491 and rs37973).

In general, it is possible to use any method with which it is possible to determine the alleles present at the rs28362491 and the rs37973 loci. As a non-limiting example, detection of these alleles may be performed by allelic discrimination. The term "allelic discrimination" is used herein in a non-limiting manner, and comprises methods such as probe hybridization, amplification, mass spectrometry, southern blotting, lateral flow assay, nucleotide incorporation, oligonucleotide ligation, invasive cleavage, enzymatic digestion, or sequencing. Preferably, the variants of the invention are detected by hybridization. The term "hybridization" as used herein refers to the formation of a specific complex between two single-stranded polynucleotide sequences due to complementary base pairing. "Specific complex formation" refers to the formation of a complex that is dependent on the precise sequence at the rs locus. During hybridization, the presence of a mismatch error at the level of the base of interest destabilizes the interaction between the sequence of interest and the complementary sequence. This destabilization can be detected. Preferably, the destabilization of the interaction prevents hybridization. As a non-limiting example, hybridization may be performed during PCR. Hybridization may also be performed following a step of amplification of the nucleic acid. Preferably, hybridization takes place between a sequence of interest, comprising an SNP, and an oligonucleotide (e.g., a probe or primer).

Preferably, the variants of the invention are detected by primer extension using nucleotide incorporation. As used herein, the expression "nucleotide incorporation" refers to the incorporation of a nucleotide that is complementary to the rs locus. The nucleotide can be modified or labeled to facilitate detection. A nucleotide may be incorporated during the sequencing of said locus, during an amplification reaction, for example by PCR, or during primer extension. As a non-limiting example, a nucleotide may be labeled with a fluorescent, chemical, magnetic or radioactive molecule. As a non-limiting example, a nucleotide may be identified by measuring its mass.

Preferably, the variants of the invention are detected by oligonucleotide ligation. As used herein, the expression "oligonucleotide ligation" refers to a ligation between two oligonucleotides, one adjacent to the other, when their complementary base pairing is perfect at the ligation site.

Preferably, the variants of the invention are detected by invasive cleavage. As used herein, the expression "invasive cleavage" refers to the formation of a cleavage-sensitive structure when overlapping probes hybridize.

Oligonucleotide ligation and invasive cleavage methods do not require a prior amplification step.

Preferably, the variants of the invention are detected by enzymatic digestion. As used herein, the expression "enzymatic digestion" refers to the digestion of a sequence by restriction enzymes that are dependent on the presence of a given variant. The restriction fragments can then be analyzed on a gel, for example by restriction fragment length polymorphism (RFLP). "Restriction fragments" refers to any fragment derived from an enzymatic digestion in which the enzyme cuts the double-stranded DNA at a specific sequence.

Preferably, the variants of the invention are detected by sequencing. "Sequencing" refers to a method for determining the sequence of a nucleic acid. A large number of sequencing methods are known in the art. For example, sequencing methods include Sanger dideoxy or chain terminated sequencing, whole genome sequencing, hybridization sequencing, pyrosequencing, capillary electrophoresis, cycle sequencing, sequencing. single base extension, solid phase sequencing, high throughput sequencing, massively parallel signature sequencing, nanopore sequencing, transmission electron microscopy sequencing, optical sequencing, mass spectrometry, 454 sequencing, labeled reversible terminator sequencing, "paired end" or "even mate" sequencing, exonuclease sequencing, ligation sequencing (e.g. according to SOLiD technology), short read sequencing, single molecule sequencing, chemical degradation sequencing, synthetic sequencing, mass parallel sequencing, real-time sequencing, semiconductor ion sequencing (e.g. Ion Torrent), multiplex sequencing of paired-end ditags (MS-PET), microfluidics, and combinations of these methods.

One approach is to use a method allowing the quantitative genotyping of nucleic acids obtained from the biological sample with a high level of precision. According to a particular embodiment, this precision is obtained by analysis of a large number of nucleic acid molecules (e.g. millions or billions) without any prior amplification step, using protocols that rely on prior knowledge of target sequences (in this case, a variant). In a preferred embodiment, the mass parallel sequencing method is used. As a non-limiting example, this method can be carried out using the "Illumina Genome Analyzer" platform, the Roche 454 platform, the ABI SOLiD platform, the Helicos single-molecule sequencing platform, the single-molecule sequencing in real time, Ion Torrent sequencing (WO 2010/008480), or nanopore sequencing. Preferably, mass parallel sequencing is performed on a random subset of nucleic acid molecules in the biological sample. Even more preferably, the method of the present invention is adapted to operate on an ABI PRISM^{®} 377 DNA sequencer, an ABI PRISM^{®} 310, 3100, 3100-Avant, 3730, or 3730x1 genetic analyzer, an Applied Biosystems SOLiD^{™} system (all from Applied Biosystems), a Genome Sequencer 20 system (Roche Applied Science), a HiSeq 2500, a HiSeq 2000, a Type IIx genomic analyzer, a MiSeq personal sequencer, a HiScanSQ (all from lllunima), the genetic analysis system including the Single Molecule Sequencer, the Analysis Engine and the Sample Loader (all from HeliScope), the Ion Proton^{™} or Ion PGM^{™} sequencer (both Ion Torrent).

In other preferred embodiments, the variants of the invention are detected by RFLP, by primer extension, by oligonucleotide ligation or by nuclease digestion.

According to another preferred embodiment, the variants of the invention are detected by probe hybridization, amplification, sequencing, mass spectrometry, Southern blotting, or by any combination of these techniques.

In a particular embodiment, the method of the invention comprises the step of amplifying nucleic acids from the biological sample, prior to detecting the variants.

As used herein, the term "nucleic acid" refers to any linear sequence of polynucleotides (e.g. of genomic DNA), such as oligonucleotides, primers, probes, amplicons, oligomeric fragments, etc. Preferably the nucleic acid is present in a biological sample from a human subject. It can be single stranded, double stranded, or a mixture of both forms. The nucleic acid may comprise coding and/or non-coding sequences. It may correspond to a fragment of an entire nucleic acid molecule. Preferably, the nucleic acid is DNA. Even more preferably, the nucleic acid is genomic DNA or a fragment thereof.

The method of the invention may contain a first step of nucleic acid isolation. As used herein, the terms "nucleic acid isolation" and "isolating nucleic acid" refer to obtaining a sample comprising the nucleic acids of a sample. Isolation may further comprise the "purification" of said nucleic acid. "Purification" refers to any process increasing the proportion of nucleic acid molecules vis-à-vis the other components of a sample, or isolating the nucleic acid molecules from other components of a sample. Purification may be partial or complete. It may comprise mechanical, enzymatic and/or chemical methods. For example, isolation may comprise a step of destabilizing a cell structure, e.g. by lysis. Isolation may also comprise a step of degrading other components, e.g. enzymatic degradation of proteins. Isolation may comprise a step of separating said nucleic acid from the other components by centrifugation, precipitation, binding to a solid support (e.g. to a silica membrane, by chromatography, to magnetic beads), organic extraction (e.g. by phenol-chloroform), etc.

As used herein, the term "amplification" refers to any process for increasing the amount of a nucleic acid molecule relative to its initial level. Amplification is dependent on the nucleic acid template and may be specific (e.g. using specific primers corresponding to exact sequences, by polymerase chain reaction (PCR)) or nonspecific (e.g. by multiple displacement amplification using hexamers). Methods for carrying out such amplification are well-known to the person skilled in the art.

In a preferred embodiment, the amplifying step is performed by PCR with primers of 12 to 30 contiguous nucleotides or by isothermal amplification or by asymmetrical PCR or by LATE (linear after the exponential) PCR of said variants such that the nucleic acid of sequence SEQ ID NO: 1 and/or 2 is amplified.

Preferably, the isothermal amplification consists of strand displacement amplification (SDA), helicase dependent amplification (HDA), loop mediated isothermal amplification (LAMP), nucleic acid sequence-based amplification (NASBA), rolling circle amplification (RCA), multiple displacement amplification (MDA) and recombinase polymerase amplification (RPA), exponential amplification reaction (EXPAR), isothermal and chimeric primer-initiated amplification of nucleic acids (ICAN), signal mediated amplification of RNA technology (SMART), nicking enzyme amplification reaction (NEAR)) and others.

More preferably, the amplification is performed by PCR with primers allowing for the amplification of a fragment of the sequence SEQ ID NO: 1 and/or 2.

### Kit of the invention

In another aspect, the present invention also concerns a kit comprising the means to detect the genotype of both the rs28362491 and the rs37973 variants in a biological sample.

As used herein, the term "kit" refers to any system for delivering materials. In the context of reaction assays, it includes systems that allow the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. The kit may further comprise a polymerase enzyme for nucleic acid amplification. The kit may comprise any appropriate buffers or written instructions.

In a preferred embodiment, said kit comprises primers or probes of 12 to 30 contiguous nucleotides that can be used for isothermal amplification or for asymmetrical PCR or for LATE (linear after the exponential) PCR of the nucleic acid of sequence SEQ ID NO: 1 and/or 2.

The term "primer" as used herein refers to an isolated nucleic acid molecule that can specifically hybridize or anneal to a 5' or 3' region of a target genomic region (plus and minus strands, respectively, or vice-versa). In general, primers are from about 10 to 30 nucleotides in length and anneal at both extremities of a region that is about 50 to 1000 nucleotides in length, more preferably about 50 to 500 nucleotides in length, more preferably about 50 to 200 nucleotides in length. Under appropriate conditions and with appropriate reagents, such primers permit the amplification of a nucleic acid molecule comprising the target nucleotide sequence (i.e. a nucleotide sequence comprising the rs locus) flanked by the primers. As they are used in pairs, they are often referred to as a "primer pair" or "primer set".

The term "probe" refers to a labeled oligonucleotide that specifically hybridizes with a nucleic acid molecule having a particular allele at the rs locus. The interaction of the probe with a particular allele at the rs locus can then be detected. As a non-limiting example, a probe may be coupled to a fluorescent, luminescent, radioactive, chemical, enzymatic, or electrical marker. The probe may comprise one or more non-natural nucleotides, e.g., a peptide nucleic acid (PNA), a peptide nucleic acid having a phosphate group (PHONA), a bridged nucleic acid or locked nucleic acid (BNA or LNA), and a morpholino nucleic acid. Non-natural nucleotides also include chemically modified nucleic acids or nucleic acid analogs such as methylphosphonate-type DNA or RNA, phosphorothioate-type DNA or RNA, phosphoramidate-type DNA or RNA, and 2'-O-methyl-type DNA or RNA. The length of a probe can be between 8 and 50 nucleotides. Preferably, the length of a probe is between 9 and 40 nucleotides. Preferably, the probe is a labeled probe of 8 to 50 contiguous nucleotides hybridizing with the two rs of the invention.

Preferably, the probe or the primer comprises a nucleotide base that is complementary to one of the alleles of the variants. In one aspect, primer hybridization may be performed in the context of the multiplex allele-specific diagnostic method (MASDA) or a DNA chip. Alternatively, the variants may be detected via probe hybridization using a molecular beacon or a hydrolysis probe (e.g. Taqman), or specific dynamic allele-specific hybridization (DASH). Preferably, hybridization is performed on a solid support. Even more preferably, hybridization is performed on a DNA chip, which may be composed of oligonucleotides, DNA, or cDNA, or on a SNP chip.

Preferably, the kit comprises at least one probe that is complementary to the A allele of rs37973 and/or at least one probe that is complementary to the G allele of the rs37973. Preferably, the kit comprises a probe that is complementary to the A allele of rs37973 because it is sufficient to use such a probe to detect bot the GA and AA genotypes of rs37973. Preferably, the kit also comprises at least one probe that is complementary to the D allele of rs28362491 and/or at least one probe that is complementary to the I allele of rs28362491. Preferably, the kit comprises a probe that is complementary to the D allele of rs28362491 and a probe that is complementary to the I allele of rs28362491, because these two probes will be necessary to detect patient having the genotype DI and exclude those having the genotype DD or II.

Preferably, the kit therefore comprises at least one probe that is complementary to the A allele of rs37973 and at least one probe that is complementary to the D allele of rs28362491 and at least one probe that is complementary to the I allele of rs28362491.

The kit of the invention may also contain a corticosteroid drug (that will be advantageously administered to the patients that carry the appropriate genotype(s)).

The kit of the invention may also contain positive reference samples containing the DNA of either homozygous (non-mutated and mutated) rs28362491, or homozygous (non-mutated and mutated) rs37973, or heterozygous rs28362491 or rs37973.

### Primers / probes

Useful primers that can be used in the method / kit of the invention are for example:
i) for detecting the rs28362491 allele:
   Sens : TTGGATCCATGCCGACCC (SEQ ID NO :3)
   Antisens : TCACTCTCTCACTTCCTGGC (SEQ ID NO :4)

Any other primers known in the art can also be used.
ii) For detecting the rs37973 allele:
Sens : ACATAAAATTCCTTGTTGACCCC (SEQ ID NO :5)
Antisens : CGGTGGGATGTTTTAGTTAGAAC (SEQ ID NO :6)

Any other primers known in the art can also be used.

The use of primers and/or probes that can specifically amplify or hybridize the genomic region of SEQ ID NO:1 and/or 2 containing rs28362491 and rs37973 respectively, or a fragment thereof, for *in vitro* predicting the efficiency of a corticosteroid treatment (e.g., in methods as defined above) is also provided herein.

In a particular aspect, the invention targets the use of primers and/or probes that can specifically amplify or hybridize the genomic region of SEQ ID NO:1 and 2 containing rs28362491 and rs37973 respectively, or a fragment thereof, for *in vitro* predicting the efficiency of a corticosteroid treatment (e.g., in methods as defined above) is also provided herein.

In a further aspect, the present invention concerns the use of the kit as defined above for implementing the prognosis method of the invention, as defined above, prior to corticosteroid administration in a patient. In particular, it concerns the use of the kit as defined above for predicting the efficiency of a corticosteroid treatment in a patient in need thereof.

### Screening methods

According to a final aspect, the present invention relates to an *in vitro* method for screening patients for responsiveness to a corticosteroid therapy, or for selecting patients that will respond to a corticosteroid therapy, said method comprising:
a) genotyping the rs37973 alleles and/or the rs28362491 alleles in a biological sample of the patients, and
b) determining the responsiveness of the patients to the corticosteroid therapy.

In this method, the presence of the alleles GA for the rs37973 variant will be indicative of a significant responsiveness to corticosteroid therapy. Similarly, the presence of the alleles DI in the rs28362491 variant will be indicative of a significant responsiveness to corticosteroid therapy. And the presence of the alleles AA or GA for the rs37973 variant and of the alleles DI in the rs28362491 variant will be also a strong indicator of a significant responsiveness to corticosteroid therapy.

Conversely, the presence of the alleles GG for the rs37973 variant or of the alleles DD or II of the rs28362491 variant will be indicative of a probable failure of the corticosteroid therapy. In this case, it will be advantageous to select an alternative treatment which does not comprise corticosteroids for the patient.

In this method, all the features defined above (nature of the sample, diseases, patients, drugs, genotyping means, etc.) apply and need not to be repeated. Also, it is obvious that the kit of the invention, as defined above, can be used for implementing this screening method as well.

### Useful associated variants

Due to population history, alleles at neighbor loci are often associated non-randomly, and said to be in linkage disequilibrium (LD). This means that certain combinations of alleles on a chromosome, also known as haplotypes, are more likely to occur together than expected from random formation of haplotypes. Consequently, alleles of variants that are in LD with a given marker having a certain effect, are likely to display a similar effect, albeit not necessarily of the same magnitude.

The term "linkage disequilibrium" or "LD" as used herein refers to the non-random association between two or more alleles at two or more loci such that certain combinations of alleles are more likely to occur together on a chromosome than other combinations of alleles that would be expected from a random formation of haplotypes from alleles based on their frequencies.

Consequently, it is possible to use in the methods and use of the invention all variants associated (or in linkage disequilibirium) with rs37973 and rs28362491 on their respective chromosomes, as these variants may display an effect on outcome dependency on hydrocortisone of the same nature as these variants and their combination.

The present inventors generated the whole-genome sequence of the investigated patients and extracted the variants in a region of 200 kbp surrounding the GLCCI1 and NFKB1 loci. They computed the pairwise r² coefficient of allelic association, an LD parameter commonly used in population genetics, of rs37973 with all other variants near GLCCI1 on the one hand, and of rs28362491 with all other variants near NFKB1 on the other hand.

Table 3 below (for rs37973) and Table 4 below (for rs28362491) list these markers (1st column) with an r² (2nd column) greater than 0.5 and the P value of the log-rank test of the influence of the hydrocortisone treatment on survival at 90-day in patients who were heterozygous (DI) for rs26382491 and homozygous for the minor allele of each of the listed markers (3rd column of Table 4) or on survival at 90-day in patients who were homozygous (AA) for rs37973 and heterozygous for each of the listed markers (3rd column of Table 3). These results show that variants other than rs37973 and rs28362491 display similar effects as rs37973 and rs28362491, as a consequence of their LD with these variants.

The present invention therefore encompasses all the methods and uses of the invention, using either the reference variants rs37973 and rs28362491 provided herein, or the associated variants thereof having a high linkage disequilibrium with these two reference variants. In the context of the present invention, two variants have a "high linkage disequilibrium" when the r² value is equal or superior to 0.5.

A non exhaustive list of associated variants for both rs37973 and rs28362491 is provided hereafter.

The 85 variants on chr 7 associated with rs37973 with a r² > 0.5 are :
rs37956, rs37957, rs37958, rs37962, rs37963, rs37964, rs37965, rs37966, rs37967, rs37968, rs37969, rs37970, rs37971, rs37972, rs37973, rs37974, rs37976, rs28177, rs37977, rs37978, rs37979, rs37983, rs37984, rs4034736, rs56070884, rs37985, rs37986, rs37987, rs3075787, rs37990, rs37991, rs37992, rs37994, rs37995, rs37996, rs37997, rs397805726, rs38000, rs38005, rs38006, rs38007, rs71014743, rs38008, rs38009, rs38010, rs38011, rs38012, rs28375221, rs62433404, rs28670736, rs38013, rs38014, rs38015, rs258850, rs258851, rs34069088, rs6463753, rs7810540, rs7796831, rs7798367, rs1476823, rs10281919, rs10238003, rs10278726, rs4725055, rs7785493, rs145936113, rs7782916, rs9655516, rs6953808, rs4725056, rs7808399, rs59475860, rs4725058, rs4725059, rs6943211, rs10278791, rs4256496, rs11981372, rs139079995, rs10264999, rs10226865, rs17568389, rs4725065, rs10253861. All these variants can be used instead of rs37973 in the methods / uses of the invention.

Among these LD variants of rs37973, particularly preferred variants are those having a P-value lower than the P-value of rs37973, namely: rs37996, rs37976, rs37979, rs37986, rs37987, rs397805726, rs37984, and rs37972. Other useful LD variants of rs37973 are those having a P-value lower than 0.001, namely: rs28177, rs37985, rs3075787, rs38007, rs38000, rs38008, rs38009, rs38015, rs258850, rs258851, rs34069088, rs7796831, rs1476823, rs4725055, rs145936113, rs4725056, rs7808399, rs59475860, rs11981372, rs10226865, rs37969 and rs37971. All these variants can be used instead of rs37973 in the methods / uses of the invention.

The 150 variants on chr 4 associated with rs28362491 with a r² > 0.5 are :
rs10019804, rs138977631, rs35910516, rs10026278, rs9996514, rs1882949, rs2085548, rs11733168, rs62328536, rs4624655, rs4698857, rs141936164, rs28882677, rs10024473, rs11097786, rs11097787, rs11097788, rs2168805, rs2125213, rs72696109, rs2903282, rs747559, rs6811263, rs6833745, rs72696114, rs28573147, rs980455, rs28362491, rs72696119, rs2272676, rs10013613, rs3774932, rs3774933, rs17032705, rs62328541, rs3774936, rs3774937, rs1120986, rs62328542, rs11934404, rs74462352, rs3774938, rs1598857, rs4235404, rs4647980, rs1599961, rs1585215, rs1585214, rs1585213, rs1598856, rs4647983, rs230535, rs170731, rs230534, rs230533, rs230532, rs230531, rs230529, rs230528, rs230527, rs230526, rs230525, rs3830689, rs3836561, rs230523, rs4648010, rs230522, rs118882, rs230521, rs230520, rs230519, rs230518, rs230517, rs230516, rs230515, rs230514, rs230512, rs230511, rs4648011, rs230509, rs230508, rs230507, rs230505, rs230504, rs230491, rs230492, rs230493, rs230494, rs230495, rs230496, rs230497, rs230498, rs230499, rs230500, rs230538, rs230539, rs230540, rs230541, rs230542, rs230543, rs230544, rs1801, rs11723120, rs11097789, rs4699030, rs1005819, rs770614407, rs759455206, rs760521269, rs76230098, rs60697656, rs61357101, rs10711129, rs1598858, rs1598859, rs17032850, rs3774956, rs4648045, rs3821958, rs3774957, rs1287, rs7667496, rs7692606, rs1020759, rs3774959, rs3774960, rs59123962, rs4698858, rs1020760, rs4648050, rs4648051, rs4648052, rs4648055, rs4648058, rs4648068, rs3774963, rs3774964, rs7684888, rs11397781, rs11722146, rs12509403, rs12509517, rs3774967, rs4699031, rs4235406, rs3755867, rs9790601, rs7683854, rs3817685, rs4648133. All these variants can be used instead of rs28362491 in the methods / uses of the invention.

Among these LD variants of rs28362491, particularly preferred variants are those having a P-value lower than the P-value of rs28362491, namely: rs3774933, rs17032705, rs74462352, rs1598857, rs1585213, rs3774938, rs1599961, rs28882677, rs10024473, rs11097787, rs2168805, rs2903282, rs747559, rs6811263, rs6833745, and rs980455.

Other useful LD variants of rs28362491 are those having a P-value lower than 0.003, namely: rs72696119, rs230507, rs230504, rs230491, rs230493, rs230535, rs170731, rs230534, rs230533, rs230532, rs230531, rs230527, rs230525, rs3836561, rs230523, rs4648010, rs230522, rs118882, rs230520, rs230519, rs230518, rs230516, rs230515, rs230514, rs230511, rs230509, rs230508, rs230492, rs230540, rs230542, rs11097789, rs1598858 and rs4648050. All these variants can be used instead of rs28362491 in the methods / uses of the invention.

These rs and the corresponding genotypes thereof can be identified in the data bank dbSNP provided by the NCBI at https://www.ncbi.nlm.nih.gov/snp/.

### Figure legends

The **figure 1** shows that the protection afforded by corticosteroids is associated with the genotypes of rs28362491 (upper panel) and rs37973 (lower panel).

The **figure 2** shows the analysis of corticosteroids effect stratified by rs28362491 (upper panel) and rs37973 (lower panel) genotypes, using the Cox-Mantel-Haenzsel test.

The **figure 3** shows the effect of corticosteroids (dark lines) versus placebo (light lines) on survival in APROCCHS patients depending on their genotype scores calculated as follows: a value of 1 was assigned to DI genotype at rs28362491 and 0 otherwise (DD or II); a value of 0 was assigned to GG genotype at rs37973, 1 to GA and 2 to AA; the genotype score was defined as the sum of both values at each locus, so that N=1 corresponds to the genotypes GA + DD or II and GG + DI; N=2 corresponds to GA + DI and AA + DD or II; N=3 corresponds to the genotype AA + DI. The shaded areas indicate the 95% confidence intervals of Kaplan-Meier estimates of survival probabilities. The P values are those of the log-rank tests. Number of patients (placebo/corticosteroids) with each genotype score: 0, 34 (13, 21); 1, 100 (51/49); 2, 142 (75,67); 3, 47 (26, 21).

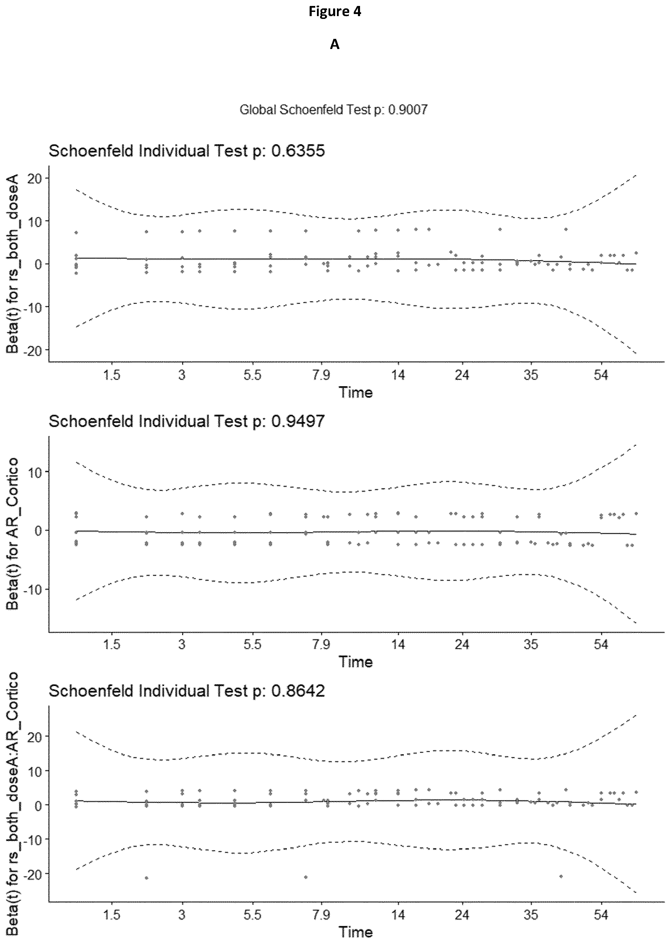

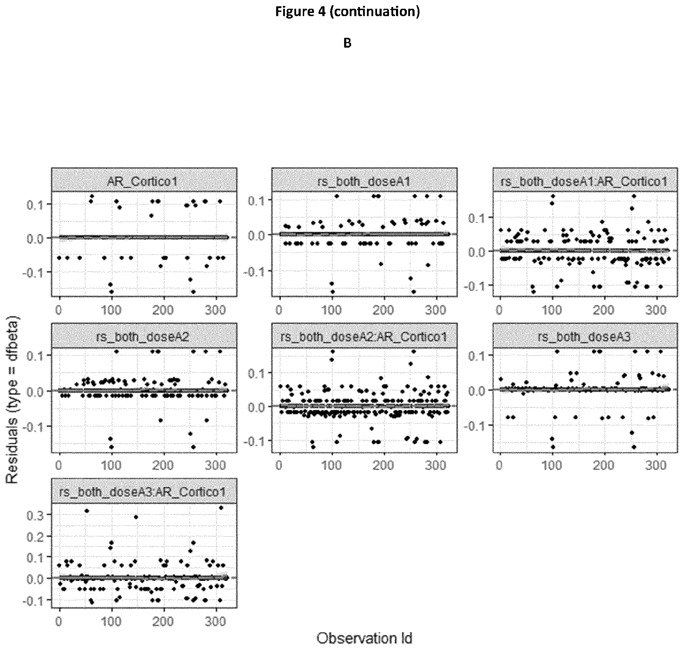

The **figure 4** shows the tests of the assumptions underlying the Cox model used to assess the link between the presence of the genotype and the corticosteroid effect: the panel A shows Schoenfeld tests of the Cox regression model (for hazards proportionality) and the panel B shows the goodness of fit of the Cox regression model.

The **figure 5** shows that there is no significant effect of corticosteroids in patients lacking the rs28362491^{∗}DI and rs27973^{∗}AA genotypes (upper panel) and that there is a detectable effect of corticosteroids in patient heterozygous for each variant, namely carrying the genotypes DI and GA (lower panel). "Protective genotypes < 3" refers to all genotypic combinations other than DI at rs28362491 and AA at rs37973.

The **figure 6** shows that there is no residual effect in these 204 patients lacking both the genotypes rs28362491^{∗}DI and rs37973^{∗}AA.

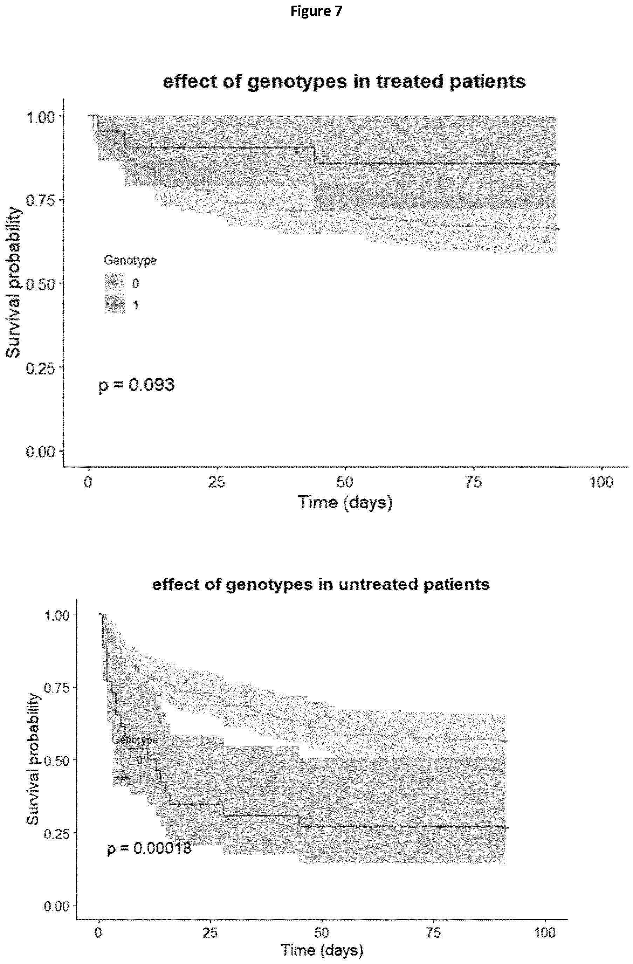

The **figure 7** shows the impact of combined rs28362491^{∗}DI and rs37973^{∗}AA genotypes (Genotype) on survival probability in 150 corticosteroids-treated (upper panel) or 166 untreated patients (lower panel) of the APROCCHS cohort. P values are those of the log-rank test.

### Examples

### Methods

### Study Design and Oversight

Information on the design and conduct of the APROCCHSS trial, including trial protocol and amendments, and statistical analysis plan, has been previously reported [12] (see online supplementary appendix). The Ethics Committee (Comité de Protection des Personnes, CPP) of Saint-Germain-en-Laye, France, approved the trial protocol including genetic analyses. Participants or their legally authorized next of kin provided written informed consent before inclusion, or deferred written informed consent was obtained from patients, as appropriate. All authors had full and independent access to all data, and vouch for the integrity, accuracy, and completeness of the data and analysis, and for the adherence to study protocol.

The trial was registered on February 19, 2008, before inclusion of the first patient, at ClinicalTrials.gov under the number NCT00625209.

### Patients

The Activated Protein C and Corticosteroids for Human Septic Shock (APROCCHSS) trial (NCT00625209) was described previously [12]. Among the 1241 patients recruited in APROCCHSS trial, frozen blood sample for genotyping was available for 325 patients.

### Interventions

Patients were randomly allocated to receive hydrocortisone+fludrocortisone or their respective placebos. Hydrocortisone was administered as a 50 mg intravenous bolus every 6 hours and fludrocortisone was given as a 50 µg tablet via a nasogastric tube once daily in the morning. Treatments were administered for 7 days without tapering. The details of study interventions are available in the full protocol in the supplementary appendix.

### Outcomes

The primary outcome was 90-day all-cause mortality. Secondary outcomes of interest included all-cause mortality at 28-day, intensive care unit and hospital length of stay.

### Genotyping

Blood samples were collected on EDTA and stored frozen. Tubes were then thawed and DNA was extracted with the Monarch^{®} genomic DNA purification kit (New England Biolabs) following the manufacturer's protocol.

The 325 patients were genotyped for two variants, one in the NFKB1 gene and the other in the GLCCI1 gene. The NFKB1 variant, rs28362491, is a deletion/insertion of 4 nucleotides located in the gene promoter, 94 base pairs before the transcription start site. The rs37973 variant is a single nucleotide variant (SNV) in the upstream region of the (GLCCI1) gene, 1000 base pairs from the transcription start site. Genotyping of both these variants was performed with the Taqman^{®} assay disclosed below as "exemplary".

### Statistical analyses

Statistical analyses of the data were performed with the R (v4.2.1) environment. Stratified tables were tested with the Cox-Mantel-Haenszel function of the Epidisplay R package. Survival analysis was conducted with the survival and survminer R packages.

### Results

Frozen blood was available for DNA extraction from 325 (159 in the corticosteroids arm and 166 in the placebo arm) of the 1241 APROCCHS patients. The clinical characteristics of these 325 patients were similar to those of the other 916 patients (not shown). There were no clinically relevant differences between treatment groups in baseline characteristics (not shown).

### Outcomes

There were 50/159 (31.4%) deaths at 90-day in the corticosteroids arm and 79/166 (47.6%) in the placebo arm (OR=0.51, 95%CI: 0.32-0.81, P=0.0032). There were 39/159 (24.5%) deaths at 28-day in the corticosteroids arm and 62/166 (37.3%) in the placebo arm (OR=0.55, 95%CI: 0.33-0.90, P=0.016). The survival probability of patients in the corticosteroids arm was significantly increased compared to that of patients in the placebo arm (P = 0.0023, not shown). The hazard ratio was significantly decreased in the corticosteroids arm whether the time-to-death variable was censored at 90-day (HR=0.58, 95% Cl: 0.41-0.83, P=) or at 28-day (HR=0.60, 95% Cl: 0.40-0.89, P=0.011). Likewise, the corticosteroid arm was associated with an increased length of stay at the hospital (37 vs 27.4 days, P = 0.028) and in the ICU (21.4 vs 16.2 days, P = 0.09).

### Genotypes x corticosteroids interaction affects mortality

The NFKB1 and GLCCI1 variants were not associated by themselves with the outcome at Day 28 or D90 (not shown). However, the combined analysis of corticosteroids treatment with the genomic markers revealed that corticosteroids effects were dependent on their genotype (Figure 1). Corticosteroids reduced the odds of dying at 90-day in patients with the heterozygous DI genotype of rs28362491 (OR=0.26, 95%CI : 0.11-0.55, P=0.00016) for the NFKB1 gene, and for the carriers of the minor A allele of rs37973 for the GLCCI1 (GA genotype, OR=0.47, 95%CI : 0.23-0.96, P=0.032 and AA genotype, OR = 0.38, 95%CI : 0.16-0.88, P=0.015). Moreover, there was a significant interaction term between the DI genotype of rs28362491 and corticosteroids treatment on the outcome at 90-day (P=0.016 for an OR = 0.32, 95%CI: 0.12-0.80)(Figure 2).

Testing a combined effect of both markers, using a partially dominant model for the A allele of rs37973 (see Methods) revealed a significant interaction term between the combined genotypes, rs28362491^{∗}DI and rs37973^{∗}AA, and corticosteroids treatment (Table 1, Figure 3, Figure 4).

**Table 1. Interaction term between genotype score and treatment arm in the APROCCHS cohort, tested by logistic regression on death at day 90 or by Cox regression on time-to-death.**

| | | Logistic regression | | Cox regression | |
|---|---|---|---|---|---|
| | | OR (95% CI) | P value | HR (95% CI) | P value |
| Arm (CS) | | | | | |
| | Placebo | - | | - | |
| | Corticosteroids | 0.72 (0.17-3.0) | | 0.74 (0.26-2.12) | |
| Genotype score (GS) | | | | | |
| | 0 | - | | - | |
| | 1 | 0.96 (0.28-3.36) | | 0.91 (0.37-2.24) | |
| | 2 | 0.82 (0.25-2.78) | | 0.80 (0.33-1.92) | |
| | 3 | 3.17 (0.8-13.4) | | 2.24 (0.89-5.62) | |
| Interaction terms | | | | | |
| | CS * GS 1 | 0.98 (0.19-5.0) | | 1.02 (0.30-3.48) | |
| | CS * GS 2 | 0.84 (0.17-4.1) | | 0.93 (0.28-3.09) | |
| | CS * GS 3 | 0.086 (0.01-0.63) | 0.019 | 0.14 (0.03-0.73) | 0.019 |

The odds of dying at 90-day and the hazard in patients from the corticosteroids arm compared to those in the placebo arm were significantly decreased only in the presence of response genotypes of both variants (OR = 0.086, 95% CI = 0.01-0.63, P = 0.019; HR = 0.14, 95% =0.03-0.73, P = 0.019; significance of the Cox regression model, 6×10⁻⁵). Conversely, there was a borderline effect (P = 0.1) of corticosteroids in patients lacking both the rs28362491^{∗}DI and rs37973^{∗}AA genotypes (Figure 5, upper panel). Nonetheless, examination of the 72 patients heterozygous for both variants (rs28362491^{∗}DI and rs37973^{∗}GA) showed an enhanced protection by corticosteroids (P = 0.044, Figure 5, lower panel). In contrast, there was no residual effect in the 204 patients lacking the rs28362491^{∗}DI genotype or carrying the homozygous rs37973^{∗}GG genotype (Figure 6).

We then focused on the group of patients carrying both the rs28362491^{∗}DI and rs37973^{∗}AA response genotypes. Those in the corticosteroids arm had an improved survival when compared with patients lacking both genotypes in the same arm (Figure 7A). However, in the placebo arm, their survival was dramatically decreased when compared with patients lacking both genotypes in the same arm (Figure 7B).

**Table.2 Statistical significance of the effect of all possible genotypic combinations at rs37973 and rs28362491 on the effect of hydrocortisone therapy on survival of sepsis patients at day 90**

| rs37973 | rs28362491 | P value |
|---|---|---|
| AA | DD | 0.5 |
| AA | DI | 5.6x10⁻⁵ |
| AA | II | 0.56 |
| GA | DD | 0.22 |
| GA | DI | 0.044 |
| GA | II | 0.7 |
| GG | DD | 0.31 |
| GG | DI | 0.93 |
| GG | II | 0.08 |

### Identification of LD variants

The whole-genome sequence of the majority (261/365) of the investigated patients was generated. Then the variants were extracted in a region of 200 kilo basepairs surrounding the GLCCI1 and NFKB1 loci. The pairwise r² coefficient of allelic association was generated for rs37973 with all other variants near GLCCI1 on the one hand, and for rs28362491 with all other variants near NFKB1 on the other hand. Table 3 for rs37973 and Table 4 for rs28362491 list these markers (1^{st} column) with an r² (2^{nd} column) greater than 0.5 (an r² equal to 1 indicates perfect LD. Conversely, an r² equal to 0 indicates no LD or allele independence).

**Table 3. Variants in LD with rs37973 (with an r² coefficient > 0.5) in a region of 200 kbp surrounding rs37973 (bold).**

| **variant** | **r²** | **Log-rank test P value** |
|---|---|---|
| rs37996 | 0.63 | 2.21E-04 |
| rs37976 | 0.76 | 3.08E-04 |
| rs37979 | 0.75 | 3.08E-04 |
| rs37986 | 0.75 | 3.08E-04 |
| rs37987 | 0.74 | 3.08E-04 |
| rs397805726 | 0.74 | 3.08E-04 |
| rs37984 | 0.53 | 3.42E-04 |
| rs37972 | 0.99 | 6.10E-04 |
| rs37973 | 1 | 6.10E-04 |
| rs28177 | 0.53 | 6.32E-04 |
| rs37985 | 0.53 | 6.32E-04 |
| rs3075787 | 0.64 | 6.50E-04 |
| rs38007 | 0.62 | 7.06E-04 |
| rs38000 | 0.69 | 8.18E-04 |
| rs38008 | 0.72 | 8.18E-04 |
| rs38009 | 0.72 | 8.18E-04 |
| rs38015 | 0.72 | 8.18E-04 |
| rs258850 | 0.69 | 8.18E-04 |
| rs258851 | 0.69 | 8.18E-04 |
| rs34069088 | 0.69 | 8.18E-04 |
| rs7796831 | 0.69 | 8.18E-04 |
| rs1476823 | 0.73 | 8.18E-04 |
| rs4725055 | 0.69 | 8.18E-04 |
| rs145936113 | 0.69 | 8.18E-04 |
| rs4725056 | 0.69 | 8.18E-04 |
| rs7808399 | 0.73 | 8.18E-04 |
| rs59475860 | 0.69 | 8.18E-04 |
| rs11981372 | 0.68 | 8.18E-04 |
| rs10226865 | 0.69 | 8.18E-04 |
| rs37969 | 0.98 | 9.25E-04 |
| rs37971 | 0.98 | 9.25E-04 |
| rs37974 | 0.6 | 0.001032575 |
| rs37990 | 0.54 | 0.001158199 |
| rs37991 | 0.54 | 0.001158199 |
| rs38005 | 0.54 | 0.001158199 |
| rs6943211 | 0.54 | 0.001158199 |
| rs6463753 | 0.68 | 0.001181956 |
| rs7810540 | 0.68 | 0.001181956 |
| rs37978 | 0.59 | 0.001429617 |
| rs37983 | 0.59 | 0.001429617 |
| rs7785493 | 0.53 | 0.001519232 |
| rs4725059 | 0.66 | 0.001530368 |
| rs10278791 | 0.7 | 0.001530368 |
| rs37992 | 0.54 | 0.001685438 |
| rs37994 | 0.54 | 0.001685438 |
| rs38010 | 0.53 | 0.001685438 |
| rs37997 | 0.58 | 0.001811055 |
| rs7798367 | 0.51 | 0.001937469 |
| rs7782916 | 0.51 | 0.001937469 |
| rs4725058 | 0.51 | 0.001937469 |
| rs37968 | 0.66 | 0.002713693 |
| rs37995 | 0.5 | 0.003233098 |
| rs71014743 | 0.5 | 0.003233098 |
| rs38011 | 0.5 | 0.003233098 |
| rs38012 | 0.5 | 0.003233098 |
| rs28375221 | 0.5 | 0.003233098 |
| rs62433404 | 0.5 | 0.003233098 |
| rs28670736 | 0.5 | 0.003233098 |
| rs38013 | 0.5 | 0.003233098 |
| rs38014 | 0.5 | 0.003233098 |
| rs4256496 | 0.6 | 0.003450997 |
| rs10264999 | 0.6 | 0.003450997 |
| rs17568389 | 0.56 | 0.003450997 |
| rs4725065 | 0.56 | 0.003450997 |
| rs10253861 | 0.61 | 0.003606786 |
| rs37977 | 0.5 | 0.00493174 |
| rs4034736 | 0.5 | 0.00493174 |
| rs56070884 | 0.5 | 0.00493174 |

**Table 4. Variants in LD with rs28362491 (with an r² coefficient > 0.5) in a region of 200 kbp surrounding rs28362491 (bold).**

| **variant** | **r²** | **Log-rank test P value** |
|---|---|---|
| rs3774933 | 0.96 | 0.001261719 |
| rs17032705 | 0.95 | 0.001261719 |
| rs74462352 | 0.95 | 0.001261719 |
| rs1598857 | 0.96 | 0.001261719 |
| rs1585213 | 0.95 | 0.001261719 |
| rs3774938 | 0.95 | 0.001840571 |
| rs1599961 | 0.95 | 0.001840571 |
| rs28882677 | 1 | 0.002386316 |
| rs10024473 | 1 | 0.002386316 |
| rs11097787 | 0.99 | 0.002386316 |
| rs2168805 | 0.99 | 0.002386316 |
| rs2903282 | 1 | 0.002386316 |
| rs747559 | 1 | 0.002386316 |
| rs6811263 | 1 | 0.002386316 |
| rs6833745 | 0.99 | 0.002386316 |
| rs980455 | 0.99 | 0.002386316 |
| **rs28362491** | 1 | 0.002386316 |
| rs72696119 | 1 | 0.002386316 |
| rs230507 | 0.73 | 0.00252044 |
| rs230504 | 0.73 | 0.00252044 |
| rs230491 | 0.73 | 0.00252044 |
| rs230493 | 0.73 | 0.00252044 |
| rs230535 | 0.71 | 0.002932217 |
| rs170731 | 0.71 | 0.002932217 |
| rs230534 | 0.7 | 0.002932217 |
| rs230533 | 0.71 | 0.002932217 |
| rs230532 | 0.71 | 0.002932217 |
| rs230531 | 0.71 | 0.002932217 |
| rs230527 | 0.7 | 0.002932217 |
| rs230525 | 0.71 | 0.002932217 |
| rs3836561 | 0.7 | 0.002932217 |
| rs230523 | 0.71 | 0.002932217 |
| rs4648010 | 0.71 | 0.002932217 |
| rs230522 | 0.71 | 0.002932217 |
| rs118882 | 0.71 | 0.002932217 |
| rs230520 | 0.7 | 0.002932217 |
| rs230519 | 0.71 | 0.002932217 |
| rs230518 | 0.71 | 0.002932217 |
| rs230516 | 0.71 | 0.002932217 |
| rs230515 | 0.71 | 0.002932217 |
| rs230514 | 0.71 | 0.002932217 |
| rs230511 | 0.7 | 0.002932217 |
| rs230509 | 0.7 | 0.002932217 |
| rs230508 | 0.71 | 0.002932217 |
| rs230492 | 0.7 | 0.002932217 |
| rs230540 | 0.69 | 0.002932217 |
| rs230542 | 0.69 | 0.002932217 |
| rs11097789 | 0.69 | 0.002932217 |
| rs1598858 | 0.69 | 0.002932217 |
| rs4648050 | 0.51 | 0.00296148 |
| rs10013613 | 0.94 | 0.003078452 |
| rs2125213 | 0.99 | 0.003447343 |
| rs4648051 | 0.55 | 0.003913405 |
| rs4648055 | 0.55 | 0.003913405 |
| rs11722146 | 0.55 | 0.003913405 |
| rs12509517 | 0.55 | 0.003913405 |
| rs4235406 | 0.55 | 0.003913405 |
| rs230529 | 0.84 | 0.004035564 |
| rs230528 | 0.84 | 0.004035564 |
| rs230526 | 0.84 | 0.004035564 |
| rs3830689 | 0.84 | 0.004035564 |
| rs230521 | 0.84 | 0.004035564 |
| rs230517 | 0.84 | 0.004035564 |
| rs230512 | 0.84 | 0.004035564 |
| rs230505 | 0.84 | 0.004035564 |
| rs230494 | 0.83 | 0.004035564 |
| rs230495 | 0.83 | 0.004035564 |
| rs230496 | 0.83 | 0.004035564 |
| rs230543 | 0.82 | 0.004035564 |
| rs230544 | 0.82 | 0.004035564 |
| rs4699030 | 0.82 | 0.004035564 |
| rs1005819 | 0.82 | 0.004035564 |
| rs770614407 | 0.82 | 0.004035564 |
| rs759455206 | 0.82 | 0.004035564 |
| rs760521269 | 0.82 | 0.004035564 |
| rs76230098 | 0.82 | 0.004035564 |
| rs60697656 | 0.82 | 0.004035564 |
| rs61357101 | 0.82 | 0.004035564 |
| rs10711129 | 0.81 | 0.004035564 |
| rs3774956 | 0.82 | 0.004035564 |
| rs3821958 | 0.82 | 0.004035564 |
| rs1287 | 0.82 | 0.004035564 |
| rs1020759 | 0.82 | 0.004035564 |
| rs3774960 | 0.82 | 0.004035564 |
| rs4698858 | 0.82 | 0.004035564 |
| rs1020760 | 0.82 | 0.004035564 |
| rs230497 | 0.68 | 0.004232917 |
| rs230499 | 0.68 | 0.004232917 |
| rs230500 | 0.68 | 0.004232917 |
| rs1801 | 0.67 | 0.004232917 |
| rs11723120 | 0.67 | 0.004232917 |
| rs1598859 | 0.67 | 0.004232917 |
| rs17032850 | 0.67 | 0.004232917 |
| rs4648045 | 0.67 | 0.004232917 |
| rs3774957 | 0.67 | 0.004232917 |
| rs7667496 | 0.67 | 0.004232917 |
| rs7692606 | 0.67 | 0.004232917 |
| rs3774959 | 0.67 | 0.004232917 |
| rs59123962 | 0.67 | 0.004232917 |
| rs62328536 | 0.72 | 0.004452409 |
| rs4624655 | 0.72 | 0.004452409 |
| rs4698857 | 0.72 | 0.004452409 |
| rs141936164 | 0.72 | 0.004452409 |
| rs11097786 | 0.72 | 0.004452409 |
| rs72696109 | 0.72 | 0.004452409 |
| rs72696114 | 0.72 | 0.004452409 |
| rs2272676 | 0.71 | 0.004452409 |
| rs62328541 | 0.71 | 0.004452409 |
| rs3774936 | 0.71 | 0.004452409 |
| rs3774937 | 0.71 | 0.004452409 |
| rs62328542 | 0.71 | 0.004452409 |
| rs4235404 | 0.71 | 0.004452409 |
| rs4647980 | 0.71 | 0.004452409 |
| rs1585215 | 0.71 | 0.004452409 |
| rs4647983 | 0.7 | 0.004452409 |
| rs230539 | 0.68 | 0.004488518 |
| rs4648011 | 0.86 | 0.004842642 |

In addition, in Table 4, is also reported the P value of the log-rank test of the influence of the hydrocortisone treatment on survival at 90-day in patients who were heterozygous (DI) for rs26382491 and homozygous for the minor allele of each of the listed markers (3^{rd} column). Likewise, in Table 3, is reported the P value of the log-rank test of the influence of the hydrocortisone treatment on survival at 90-day in patients who were homozygous (AA) for rs37973 and heterozygous for each of the listed markers (3^{rd} column). For both tables, output was filtered for P values less than 0.005 and ordered by increasing P value.

These findings thus support that other variants than rs37973 and rs28362491 display similar effects as observed for rs37973 and rs28362491, by consequence of their LD with these variants.

### Exemplary genotyping protocol

This protocol is intended for the genotyping from whole blood of variants in the promoters of the NFKB1 and GLCCI1 genes. It takes place in two stages. First, the sequence around the variant is amplified from the blood. Then, the variants are genotyped with the TaqMan^{®} method.

The MyTaq^{™} Blood-PCR kit (ref: BIO-25054) sold by meridian Bioscience can be used. The MyTaq^{™} Blood-PCR Kit is a ready-to-use optimized mix for rapid and highly specific direct PCR from whole blood samples collected with various anticoagulants (EDTA, citrate, heparin) of human and non-human origin. The MyTaq^{™} Blood-PCR Kit was specifically developed to overcome PCR inhibitors commonly found in blood samples, to significantly increase PCR sensitivity and success rates. The speed and high specificity of the MyTaq^{™} Blood-PCR Kit also makes it very suitable for multiplex PCR applications.

### 1st step: PCR from whole blood

The following components have to be prepared in a DNAse-free reaction tube. When analyzing multiple blood samples, MyTaq Blood-PCR, primers and water are mixed to reduce pipetting errors. The master mix can then be aliquoted into each reaction tube and the blood samples added at the end.

| **Reagents¹** | **Volume/tube** |
|---|---|
| MyTaq Blood-PCR Mix (2X) | 12,5 µι. |
| Sens primer (10 µM) | 0,5 µι. |
| Antisens primer (10 µM) | 0,5 µL |
| Nuclease-free water | 10,5 µL |
| **Volume of the mix = 24 µL** | |
| Blood ² | 1 µL |
| **Final volume of the reaction = 24 µl + 1 µl = 25 µl** | |

| | |
|---|---|
| ¹ The MyTaq Blood-PCR kit and primers are stored at -20°C. Nuclease-free water should be purchased commercially and stored at room temperature. ² Frozen whole blood were used. Thawing was done either in lukewarm water or at room temperature. There is no need to boil the blood tubes. | |

The PCR primers are :
i) for detecting the rs28362491 allele:
   Sens : TTGGATCCATGCCGACCC (SEQ ID NO :3)
   Antisens : TCACTCTCTCACTTCCTGGC (SEQ ID NO :4)
ii) For detecting the rs37973 allele:
   Sens : ACATAAAATTCCTTGTTGACCCC (SEQ ID NO :5)
   Antisens : CGGTGGGATGTTTTAGTTAGAAC (SEQ ID NO :6)

### Characteristics of the PCR program :

| **Step :** | **Température** | **Time** | **Cycles** |
|---|---|---|---|
| Initial denaturation | 95°C | 3 minutes | 1 |
| denaturation | 95°C | 15 seconds | 20 |
| Hybridation | 55°C | 15 seconds | |
| Extension | 72°C | 45 seconds | |
| Final | 4°C | ∞ | |

The duration of the PCR was of about 1h15'.

### 2^{nd} step: genotyping with the TaqMan^{®} method

The following components can be prepared in a DNAse-free reaction tube. For the analysis of several amplicon samples, it is necessary to mix MyTaq Blood-PCR, the TaqMan^{®} probe and water in order to reduce pipetting errors. The master mix can then be aliquoted into each reaction tube and the blood samples added at the end.

| **Reagents ¹** | **Volume/tube** |
|---|---|
| MyTaq Blood-PCR Mix (2X) | 5 µι. |
| TaqMan^{®} probe (40X) | 0,5 µL |
| Nuclease-free water | 3,5 µL |
| **Volume of the mix = 9 µL** | |
| Amplicon² | 1 µL |
| **Final volume of the reaction = 9 µl + 1 µl = 10 µl** | |

| | |
|---|---|
| 1 MyTaq Blood-PCR kit and TaqMan^{®} probe are stored at -20°C. Nuclease-free water should be purchased commercially and stored at room temperature. 2 We used frozen whole blood. Thawing was done either in lukewarm water or at room temperature. There is no need to boil the blood tubes. | |

### Characteristics of the TaqMan program :

| **Step** | **Température** | **Time** | **Cycles** |
|---|---|---|---|
| Initial denaturation | 95°C | 3 minutes | 1 |
| Denaturation | 95°C | 15 seconds | 30 |
| Hybridation | 55°C | 15 seconds | |
| Extension | 72°C | 45 seconds | |
| Reading | 55°C | 1 minute | 1 |

Estimated duration of the PCR: 1h15'

The results can be analyzed using the CFX Manager software. It is free and provided by Biorad (can be downloaded from the Biorad website).

It is advantageous to put on the x-axis the fluorescence of one allele and on the y-axis the fluorescence of the other, so that:
- The H₂0 control will be at the bottom left i.e., without fluorescence (because no DNA)
- the heterozygotes will be at the top right i.e., the fluorescences of two alleles (mutated allele and non-mutated allele).
- The non-mutated homozygotes will at the top left i.e., the fluorescence of a single allele (unmutated allele).
- The mutated homozygotes will be at the bottom right i.e., the fluorescence of a single allele (mutated allele).

### Bibliographic references

1. Singer M, Deutschman CS, Seymour CW, et al. The Third International Consensus Definitions for Sepsis and Septic Shock (Sepsis-3). JAMA 2016;315(8):801-10.
2. Cohen J. The immunopathogenesis of sepsis. Nature 2002;420(6917):885-91.
3. World Health Organization. Global report on the epidemiology and burden of sepsis: current evidence, identifying gaps and future directions [Internet]. Geneva: World Health Organization; 2020 [cited 2022 Jul 15]. Available from: https://apps.who.int/iris/handle/10665/334216
4. Reinhart K, Levy MM, Finfer SS, et al. Reducing the global burden of sepsis: a positive legacy for the COVID-19 pandemic? Intensive Care Med 2021;47(7):733-6.
5. Dupuis C, Bouadma L, Ruckly S, et al. Sepsis and septic shock in France: incidences, outcomes and costs of care. Annals of Intensive Care 2020;10(1):145.
6. Barnes PJ, Adcock IM. Glucocorticoid resistance in inflammatory diseases. 2009;373:13.
7. Quax RA, Manenschijn L, Koper JW, et al. Glucocorticoid sensitivity in health and disease. Nat Rev Endocrinol 2013;9(11):670-86.
8. Annane D, Renault A, Brun-Buisson C, et al. Hydrocortisone plus Fludrocortisone for Adults with Septic Shock. N Engl J Med 2018;378(9):809-18.
9. Group TRC. Dexamethasone in Hospitalized Patients with Covid-19 - Preliminary Report. New England Journal of Medicine [Internet] 2020 [cited 2020 Oct 26];Available from: https://www-nejm-org.proxy.insermbiblio.inist.fr/doi/10.1056/NEJMoa2021436
10. Schäfer ST, Gessner S, Scherag A, et al. Hydrocortisone Fails to Abolish NF-κB1 Protein Nuclear Translocation in Deletion Allele Carriers of the NFKB1 Promoter Polymorphism (-94ins/delATTG) and Is Associated with Increased 30-Day Mortality in Septic Shock. PLoS ONE 2014;9(8):e104953.
11. Tantisira KG, Lasky-Su J, Harada M, et al. Genomewide Association between GLCCI1 and Response to Glucocorticoid Therapy in Asthma. N Engl J Med 2011;365(13):1173-83.
12. Annane D, Buisson CB, Cariou A, et al. Design and conduct of the activated protein C and corticosteroids for human septic shock (APROCCHSS) trial. Annals of Intensive Care 2016;6(1):43.

## Claims

1. Corticosteroids for use for treating a patient suffering from a disease treatable by corticosteroids, wherein said patient carries the genotype DI of the rs28362491 variant located in the *NFKB1* gene and the genotype GA or AA in the rs37973 variant located in the promoter of the *GLCCI1* gene, or the associated variants thereof listed in Table 4 and Table 3 respectively, having a high linkage disequilibrium (LD) with rs28362491 and rs37973 variants.

2. Corticosteroids for use according to claim 1, wherein said patient carries the genotype DI in rs28362491 and the genotype AA in rs37973 or the respective LD variants thereof.

3. Corticosteroids for use according to claim 1 or 2, wherein said disease is chosen in the group consisting of allergies and atopy, inflammatory conditions, and cancer; said disease being preferably severe asthma or sepsis.

4. Corticosteroids for use according to any of claims 1 to 3, wherein said corticosteroid is chosen in the group consisting of : hydrocortisone, hydrocortisone acetate and other esters, cortisone acetate, tixocortol pivalate, prednisolone, methylprednisolone, prednicarbate, prednisone, amcinonide, budesonide, desonide, fluocinolone acetonide, fluocinonide, flunisolide, fludroxycortide, halcinonide, deflazacort, ulobetasol, ciclesonide, triamcinolone acetonide, beclomethasone, betamethasone, dexamethasone, fluocortolone, halometasone, paramethasone, mometasone, alclometasone dipropionate, betamethasone dipropionate, betamethasone valerate, clobetasol propionate, clobetasone butyrate, fluprednidene acetate, and mometasone furoate, or any other hydrocortisone, acetonide, or betamethasone derivatives.

5. *In vitro* method for predicting the efficiency of a corticosteroid treatment, said method comprising the step of detecting the genotype of both the rs28362491 variant and of the rs37973 variant or the associated variants thereof having a high linkage disequilibrium with these reference variants, in a biological sample of a patient in need thereof, wherein it is concluded that the corticosteroid treatment will be efficient in said patient:
- if the rs28362491 variant is of the DI genotype, and
- if the rs37973 variant is of the AA or GA genotype,
- or the associated variants thereof having a high linkage disequilibrium with these reference variants.

6. *In vitro* method of claim 5, wherein it is concluded that the corticosteroid treatment will be efficient in said patient if the rs28362491 variant is of the DI genotype and if the rs37973 variant is of the AA genotype or the respective LD variants thereof.

7. The method of claim 5 or 6, wherein said patient suffers from a disease chosen in the group consisting of allergies and atopy, inflammatory conditions, and cancer, wherein said disease is preferably severe asthma or sepsis.

8. The method of claim 5 or 6 or 7, wherein said corticosteroid is chosen in the group consisting of: hydrocortisone, hydrocortisone acetate and other esters, cortisone acetate, tixocortol pivalate, prednisolone, methylprednisolone, prednicarbate, prednisone, amcinonide, budesonide, desonide, fluocinolone acetonide, fluocinonide, flunisolide, fludroxycortide, halcinonide, deflazacort, ulobetasol, ciclesonide, triamcinolone acetonide, beclomethasone, betamethasone, dexamethasone, fluocortolone, halometasone, paramethasone, mometasone, alclometasone dipropionate, betamethasone dipropionate, betamethasone valerate, clobetasol propionate, clobetasone butyrate, fluprednidene acetate, and mometasone furoate, or any other hydrocortisone, acetonide, or betamethasone derivatives.

9. The method of any one of claims 5 to 8, wherein said biological sample is a whole blood or plasma or serum or saliva or hair or urine sample.

10. The method of any one of claims 5 to 9, further comprising the step of amplifying the nucleic acids from the biological sample, prior to detecting the variants.

11. The method of any one of claims 5 to 10, wherein amplifying the variants is performed by PCR with primers of 12 to 30 contiguous nucleotides or by isothermal amplification or by asymmetrical PCR or by LATE (linear after the exponential) PCR of said variants such that the nucleic acid of sequence SEQ ID NO: 1 and/or 2 is amplified.

12. The method of any one of claims 5 to 11, wherein the variant(s) is (are) detected by allelic discrimination, preferably by probe hybridization, amplification, sequencing, mass spectrometry, Southern blotting, lateral flow assay or two or more thereof.

13. Kit comprising the means to detect the genotype of both the rs28362491 and the rs37973 variants, or the respective LD variants thereof, in a biological sample.

14. The kit of claim 13, comprising primers of 12 to 30 contiguous nucleotides or by isothermal amplification or by asymmetrical PCR or by LATE (linear after the exponential) PCR of said variants such that the nucleic acid of sequence SEQ ID NO: 1 and/or 2.

15. Use of the kit as defined in claim 13 or 14, for implementing the method of claims 5 to 12 prior to corticosteroid administration in a patient.
